# EUROPÄISCHE PATENTSCHRIFT

(11) **EP 1 210 564 B1**
(45) Veröffentlichungstag und Bekanntmachung des Hinweises auf die Patenterteilung: **23.08.2006**
(21) Anmeldenummer: 01960112.9
(22) Anmeldetag: 06.07.2001
(51) Int. Cl.: G01B 11/24, G01B 9/02

(54) **INTERFEROMETRISCHE MESSVORRICHTUNG**
INTERFEROMETRIC MEASURING DEVICE
DISPOSITIF DE MESURE INTERFEROMETRIQUE

(30) Priorität: 07.07.2000 DE 10033028; 03.07.2001 DE 10131779
(43) Veröffentlichungstag der Anmeldung: 05.06.2002
(73) Patentinhaber: ROBERT BOSCH GMBH, 70442 Stuttgart (DE)
(72) Erfinder: PRINZHAUSEN, Friedrich, 73732 Esslingen (DE); LINDNER, Michael, 71397 Leutenbach (DE); THOMINET, Vincent, CH-1110 Morges (CH)
(86) Internationale Anmeldenummer: PCT/DE2001/002517
(87) Internationale Veröffentlichungsnummer: WO 2002/004888

(56) Entgegenhaltungen:
- EP-A- 0 534 795
- WO-A-01/38820
- DE-A- 19 808 273
- DE-C- 19 721 843
- US-A- 5 321 501

## Beschreibung

Die Erfindung bezieht sich auf eine interferometrische Messvorrichtung, die zur Formvermessung eines mehrere Flächen aufweisenden Objektes ausgebildet ist, mit einer eine kurzkohärente Strahlung abgebenden Strahlungsquelle, einem Strahlteiler, mit dem ein über einen Objektlichtweg zu dem Objekt geleiteter Objektstrahl und ein über einen Referenzlichtweg zu einer reflektierenden Referenzebene geleiteter Referenzstrahl erzeugbar sind, und mit einem Bildwandler, der die von den Flächen und der Referenzebene zurückgeworfene und zur Interferenz gebrachte Strahlung aufnimmt und einer Auswerteeinrichtung zum Bestimmen eines die Flächen betreffenden Messergebnisses zuführt, wobei für die Messung die optische Länge des Objektlichtweges relativ zu der optischen Länge des Referenzlichtweges änderbar ist und die Messung der Flächen unter relativer Änderung der optischen Länge des Objektlichtweges zu der optischen Länge des Referenzlichtweges erfolgt.

Eine derartige interferometrische Messvorrichtung, bei der allerdings mit getrennten Messstrahlen beaufschlagte Photodetektoren vorhanden sind, ist in der DE 197 21 843 C1 angegeben. Bei dieser bekannten Messvorrichtung wird eine von einer Strahlungserzeugungseinheit abgegebene kurzkohärente Strahlung mittels eines Strahlteilers in einen Referenzstrahl und einen Objektstrahl aufgeteilt, der über einen polarisierenden Doppelstrahlteiler in zwei Objekt-Teilstrahlen mit unterschiedlicher Polarisationsrichtung zum Vermessen zweier Flächen des Objekts sowie einen zu einem Spiegel geführten, zur Kalibrierung verwendeten Bezugsstrahl aufgeteilt wird. Der in dem vorgelagerten Strahlteiler gebildete Referenzstrahl wird zum Ändern seiner optischen Weglänge über elektrisch angesteuerte elektrooptische Deflektoren auf ein reflektierendes Element in Form eines Beugungsgitters gelenkt. Die von den beiden Flächen des Objektes reflektierten, mit dem Referenzstrahl interferierenden Strahlanteile werden mittels einer Polarisationsvorrichtung getrennt und über zwei verschiedene Wege den beiden Photodetektoren zugeführt, die die von den beiden Flächen des Objektes reflektierten Messstrahlen aufnehmen, um z.B. den Durchmesser einer Bohrung zu bestimmen.

Bei einer in der DE 198 08 273 A1 offenbarten interferometrischen Messvorrichtung mit kurzkohärenter Strahlung ist die Ausbildung derart, dass eine Objektfläche in einem engen Hohlraum des Objektes vermessen werden kann.

Eine weitere interferometrische Messvorrichtung ist in der DE 41 08 944 A1 angegeben. Bei dieser bekannten interferometrischen Messvorrichtung, die auf dem Messprinzip der sogenannten Weisslichtinterferometrie oder Kurzkohärenzinterferometrie beruht, gibt eine Strahlungsquelle kurzkohärente Strahlung ab, die über einen Strahlteiler in einen ein Messobjekt beleuchtenden Objektstrahl und einen eine reflektierende Referenzebene in Form eines Referenzspiegels beleuchtenden Referenzstrahl aufgeteilt wird. Um die Objektoberfläche in Tiefenrichtung abzutasten, wird der Referenzspiegel mittels eines Piezostellelementes in Richtung der optischen Achse des Referenzlichtwegs verfahren. Wenn der Objektlichtweg und der Referenzlichtweg übereinstimmen, ergibt sich im Bereich der Kohärenzlänge ein Maximum des Interferenzkontrastes, der mittels eines photoelektrischen Bildwandlers und einer nachgeschalteten Auswerteeinrichtung erkannt und zur Bestimmung der Kontur der Objektoberfläche auf der Grundlage der bekannten Auslenkposition des Referenzspiegels ausgewertet wird.

Weitere derartige interferometrische Messvorrichtungen bzw. interferometrsiche Messverfahren auf der Basis der Weisslichtinterferometrie sind in P. de Groot, L. Deck, "Surface profiling by analysis of white-light interferograms in the spatial frequency domain" J. Mod. Opt., Vol. 42, No. 2, 389-401, 1995 und Th. Dresel, G. Häusler, H. Venske; "Three-dimensional sensing of rough surfaces by coherence radar", Appl. Opt., Vol. 31, No. 7, 919-925, 1992 angegeben.

Bei einer in der (nicht vorveröffentlichten) WO 01/38820 A1 angegebenen weiteren interferometrischen Messvorrichtung zur Formvermessung mehrerer Flächen eines Objekts, die ebenfalls auf dem Prinzip der Kurzkohärenz-Interferometrie beruht, sind im Objektlichtweg zwei Messstrahlen unterschiedlicher optischer Weglänge erzeugt, die mit einem im Referenzlichtweg reflektierten Referenzstrahl interferierend zusammenwirken und mittels einer Detektionsvorrichtung aufgrund unterschiedlicher optischer Eigenschaften der Messstrahlen oder einer Veränderung des Umwegs erfasst werden. Als Anwendung ist die Messung an verschiedenen Oberflächen des Auges eingehender beschrieben.

Bei den bekannten interferometrischen Messvorrichtungen bzw. Messverfahren bestehen zudem Schwierigkeiten, wenn die Messaufgabe die Abtastung mehrerer von einander getrennter Flächen erfordert, die z.B. mehrere Millimeter beabstandet und/oder schräg zueinander orientiert sind.

Der Erfindung liegt die Aufgabe zugrunde, eine interferometrische Messvorrichtung der eingangs genannten Art bereit zu stellen, mit der mindestens zwei voneinander räumlich getrennte Flächen mit möglichst geringem Aufwand mit genauen, gut reproduzierbaren Messergebnissen ohne neue Justierung des Objektlichtweges vermessen werden können.

Diese Aufgabe wird mit den Merkmalen der Ansprüche 1 und alternativ 3 gelöst. Hiemach ist vorgesehen, dass in dem Objektlichtweg eine Superpositionsoptik mit einer Multifokaloptik oder einer Freie-Segmente-Optik aus verschiedenen Abbildungselementen vorgesehen ist, dass mit der Superpositionsoptik gleichzeitig von den beiden Flächen ein Bild erzeugbar ist, die über mindestens eine Zwischenabbildung im Objektlichtweg auf dem Bildwandler abgebildet werden. Alternativ ist vorgesehen, dass in dem Objektlichtweg eine Abbildungsoptik mit einer Schärfentiefe von mindestens dem optischen Wegunterschied der beiden Flächen vorgesehen ist, mit der gleichzeitig außer von der einen Fläche von der mindestens einen davor oder dahinter liegenden parallelen weiteren Fläche - oder über optische Ablenkelemente schräg oder rechtwinklig zueinander angeordneten Flächen - ein Bild erzeugbar ist, das über mindestens eine Zwischenabbildung im Objektlichtweg auf dem Bildwandler abgebildet wird.

Mit diesen Maßnahmen wird ohne neue Justierung des Objektlichtweges eine genaue Messung der unterschiedlichen Flächen ermöglicht. Zur Erfassung des Interferenzmaximums müssen lediglich die optischen Längen des Referenzlichtweges und des Objektlichtweges entsprechend den Lagen der verschiedenen Flächen nacheinander eingestellt werden. Die Freie-Segmente-Optik lässt sich dabei z.B. auch leicht an schräg zueinander gestellte oder gegenüberliegende Flächen anpassen. Mit der Multifokaloptik und auch mit der eine Schärfentiefe von mindestens dem optischen Wegunterschied der beiden Flächen aufweisenden Abbildungsoptik lassen sich unterschiedlich weit voneinander entfernte und unterschiedlich zueinander orientierte Flächen und auch z.B. deren Parallelität oder Planheit, Dicke und Durchmesser vermessen.

Verschiedene günstige Ausgestaltungen bestehen weiterhin darin, dass der Objektlichtweg zum Erzeugen eines gemeinsamen Zwischenbildes der Fläche und des Zwischenbildes der weiteren Fläche(n) in einer gemeinsamen Zwischenbildebene im Objektlichtweg ausgebildet ist und dass das gemeinsame Zwischenbild direkt oder über mindestens eine Zwischenabbildung auf dem Bildwandler abgebildet wird. Mit mindestens einer Zwischenabbildung ist zum einen eine Zwischenbildabtastung und zum anderen eine erhöhte laterale Auflösung möglich.

Eine Messung mit relativ großer lateraler Auflösung auch in engen Hohlräumen lässt sich einfach durchführen, wenn vorgesehen ist, dass der Objektlichtweg als Endoskop ausgebildet ist.

Für eine genaue Messung sind weiterhin die Maßnahmen vorteilhaft, dass zur Beleuchtung des Objektes mit einer ebenen Welle ein Lichtwellenleiter vorgesehen ist, deren objektseitiger Ausgang in eine telezentrische Abbildungsanordnung des Objektlichtweges gelegt ist, oder dass ein Beleuchtungslichtweg mit zusätzlichen Linsen und Ablenkelementen gebildet ist.

Die Messung wird dadurch ermöglicht oder weiterhin begünstigt, dass der Referenzlichtweg dem Objektlichtweg ähnliche oder identische Optiken aufweist, durch welche die Erzeugung der Interferenzen ermöglicht wird oder der Interferenzkontrast optimiert wird oder optische Einflüsse der Komponenten im Objektlichtweg kompensiert werden.

Vielfältige Möglichkeiten, auf einfache Weise verschiedene Oberflächen auch an schwer zugänglichen Stellen zu vermessen, ergeben sich dadurch, dass im Objektlichtweg eine bezüglich des Objekts starre Optik angeordnet ist und dass der starren Optik eine in Richtung ihrer optischen Achse bewegliche Optik folgt.

Eine günstige Ausbildung für den Aufbau und die Handhabung besteht darin, dass die starre Optik Teil der Superpositionsoptik ist.

Zum Erreichen einer gegen laterale Relativbewegung des Objektes robusten Messung ist es vorteilhaft vorgesehen, dass die starre Optik nach Unendlich abbildet.

Eine vorteilhafte Ausgestaltung der Erfindung besteht desweiteren darin, dass die starre Optik als Superpositionsoptik ausgebildet ist, mit der mindestens ein zum Objekt starres Zwischenbild erzeugt wird, und dass als bewegliche Optik eine im Strahlengang hinter dem starren Zwischenbild folgende Objektiv-Optik in Richtung ihrer optischen Achse beweglich zur Abtastung des normal zu dieser Achse ausgerichteten Zwischenbilds in Tiefenrichtung und Abbilden desselben direkt oder über eine oder mehrere Zwischenabbildungen auf dem Bildwandler ausgebildet ist. Durch die Erzeugung des z.B. im Objektlichtweg liegenden starren Zwischenbilds der Objektoberfläche mit der Superpositionsoptik in dem Objektlichtweg wird auch in engen Kanälen oder Bohrungen die zu messende Objektoberfläche mit relativ großer lateraler Auflösung erfassbar und mit dem Bildwandler und der nachgeschalteten Auswerteeinrichtung hinsichtlich der Tiefenstruktur auswertbar. Die Abtastung des starren Zwischenbildes ist mit relativ einfachen Maßnahmen möglich, da zu seiner Tiefenabtastung nur wenige optische Komponenten des Objektlichtweges bewegt werden müssen, wobei die jeweils abgetastete Tiefe des starren Zwischenbildes stets im Schärfetiefenbereich der beweglichen Objektivoptik bleibt, da durch die Tiefenabtastung (Tiefenscan) die Objektebene der bewegten Objektivoptik gleichsam durch das starre Zwischenbild hindurch bewegt wird und auf diese Weise z.B. die Interferenzmaxima im Bereich größter Schärfentiefe ausgewertet werden.

Die Abbildungsqualität und Genauigkeit der Auswertung wird dadurch begünstigt, dass die Zwischenabbildung für alle im Zwischenbild abgebildeten Objektpunkte gleichen Abbildungsmaßstab besitzt. Beispielsweise kann dabei der Aufbau derart sein, dass die starre Optik als 4f-Anordnung ausgebildet ist.

Bezüglich der Ausbildung der starren Optik und der beweglichen Optik sei ergänzend auf die deutsche Patentanmeldung Nr. 101 15 524 derselben Anmelderin hingewiesen.

### Zeichnung

Die Erfindung wird nachfolgend anhand von Ausführungsbeispielen unter Bezugnahme auf die Zeichnungen näher erläutert. Es zeigen:
- Fig. 1: eine schematische Darstellung einer interferometrischen Messvorrichtung nach dem Prinzip der Weisslichtinterferometrie (Kurzkohärenzinterferometrie) mit einer Freie-Segmente-Optik, wobei die Freie-Segmente-Optik in zwei um 90° zueinander gedrehten Lagen dargestellt ist,
- Fig. 2: ein weiteres Ausführungsbeispiel der interferometrischen Messvorrichtung, wobei in dem Objektlichtweg eine Superpositionsoptik mit getrennten Linsenelementen gebildet ist,
- Fig. 3: ein weiteres Ausführungsbeispiel für eine interferometrische Messvorrichtung, wobei in dem Objektlichtweg eine Bifokaloptik angeordnet ist,
- Fig. 4: ein weiteres Ausführungsbeispiel einer interferometrischen Messvorrichtung, bei dem die Strahlung in dem Referenzlichtweg und dem Objektlichtweg mit Lichtwellenleitern geführt wird und
- Fig. 5: ein weiteres Ausführungsbeispiel der interferometrischen Messvorrichtung, bei dem die Strahlung in dem Objektlichtweg über einen Beleuchtungslichtweg mit Linsen und Ablenkelementen geführt wird.

### Ausführungsbeispiel

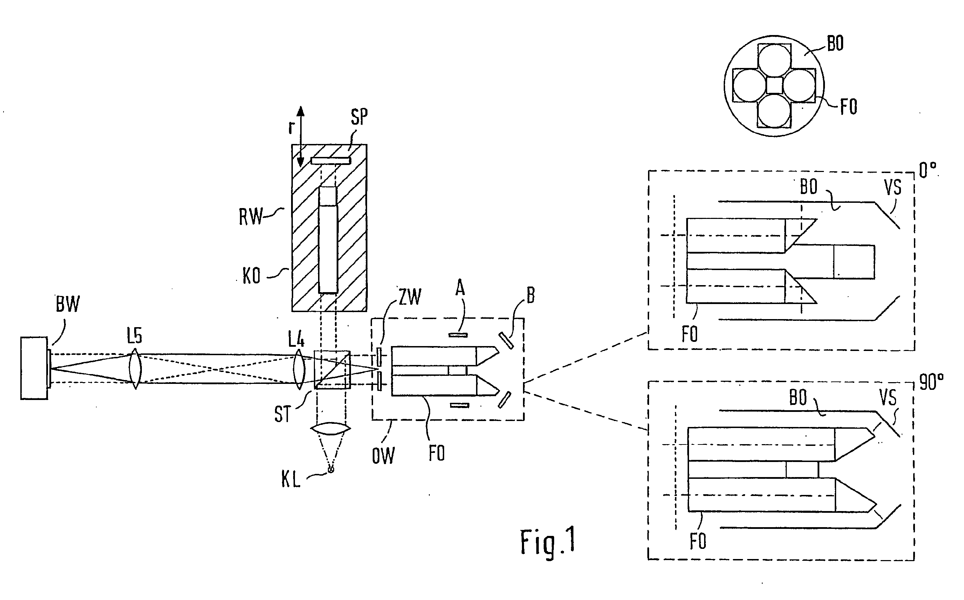

Wie Fig. 1 zeigt, weist eine auf dem Prinzip der Weisslichtinterferometrie (Kurzkohärenzinterferometrie) beruhende interferometrische Messvorrichtung einen Objektlichtweg OW, einen Referenzlichtweg RW und einen Bildwandler BW mit nachgeschalteter Auswerteeinrichtung auf, wie an sich bekannt und in den einleitend genannten Druckschriften sowie darin genannter Literatur näher beschrieben. Dabei wird ausgenutzt, dass Interferenz nur im Bereich der Kohärenzlänge auftritt, wodurch eine einfache Abstimmung der optischen Weglängen des Referenzlichtweges RW und des Objektlichtweges OW sowie z.B. die Erfassung des Interferenzmaximums ermöglicht wird. Eine von einer kurzkohärenten Lichtquelle KL abgegebene Strahlung hat dabei z.B. eine Kohärenzlänge in der Größenordnung von 10 µm. Die Strahlung der kurzkohärenten Lichtquelle KL wird mittels eines Strahlteilers ST in einen über den Referenzlichtweg RW geführten Referenzstrahl und einen über den Objektlichtweg OW geführten Objektstrahl aufgeteilt. In dem Lichtweg zu dem Bildwandler BW sind für die Abbildung vorliegend eine vierte und eine fünfte Linse L4, L5 angeordnet.

Die Messung wird dadurch ermöglicht oder weiterhin begünstigt, dass der Referenzlichtweg dem Objektlichtweg ähnliche oder identische Optiken aufweist, durch welche die Erzeugung der Interferenzen ermöglicht wird oder der Interferenzkontrast optimiert wird oder optische Einflüsse der Komponenten im Objektlichtweg kompensiert werden.

In dem Objektlichtweg OW ist als weitere Besonderheit eine Superpositionsoptik in Form einer Freie-Segmente-Optik FO angeordnet, die in den rechts daneben gezeigten Darstellungen im Querschnitt (obere Darstellung) in einer O°-Ansicht (mittlere Darstellung) und in einer 90°-Ansicht (untere Darstellung) in einem in eine Ventilbohrung BO bis in die Nähe eines Ventilsitzes VS geführten Zustand wiedergegeben ist. Mit der Freien-Segmente-Optik FO können gleichzeitig mehrere voneinander getrennte Flächen A, B der Bohrung BO bzw. des Ventilsitzes VS erfasst und in einem gemeinsamen Zwischenbild ZW in einer Zwischenbildebene im Objektlichtweg abgebildet werden, die senkrecht zu einer optischen Hauptachse des Objektlichtweges OW liegt. Die Freie-Segmente-Optik FO besitzt mehrere Licht ablenkende Flächen und abbildende Linsenelemente und ist an die jeweilige Messanforderung angepasst. Insbesondere können unterschiedlich weit von dem gemeinsamen Zwischenbild ZW entfernte und auch schräg zueinander gerichtete oder gegenüberliegende Flächen A, B erfasst und in dem gemeinsamen Zwischenbild ZW abgebildet werden.

Die Erfassung der den beiden Flächen A, B entsprechenden Interferenzmaxima erfolgt durch Änderung des Referenzlichtweges RW entsprechend einer Abtastrichtung r. Die bewegte Einheit ist strichliert dargestellt.

Die in dem Objektlichtweg OW angeordnete Superpositionsoptik weist zwei parallel geschaltete Linsen, nämlich eine erste Linse L1 und eine zweite Linse L2 mit verschiedenen Brennweiten auf, denen prismenförmige Elemente vorgeschaltet sein können. Der Objektlichtweg ist außerdem zum Erzeugen einer telezentrischen Abbildung ausgelegt. Mit den beiden Linsen L1 und L2 werden unterschiedlich weit, z.B. einige µm bis über 1 cm voneinander entfernte, parallel zueinander und senkrecht zur optischen Hauptachse des Objektlichtweges OW liegende Flächen A, B in das gemeinsame Zwischenbild ZW aus dem Zwischenbild ZA der Fläche A und dem Zwischenbild ZB der Fläche B in einer Zwischenbildebene im Objektlichtweg abgebildet. Die Brennweiten der ersten und der zweiten Linse L1, L2 sind mit F_{A}, F_{B} angegeben. In dem Strahlengang des Objektlichtweges OW ist weiterhin eine dritte Linse L3 zur Abbildung angeordnet. Zum Erfassen des Interferenzmaximums wird der Spiegel SP in Abtastrichtung r bewegt.

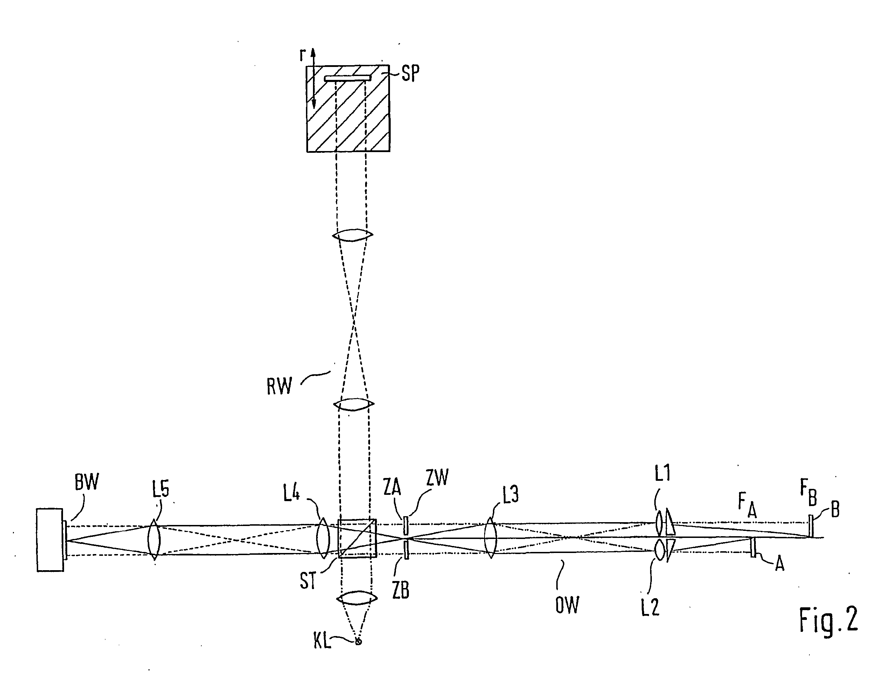

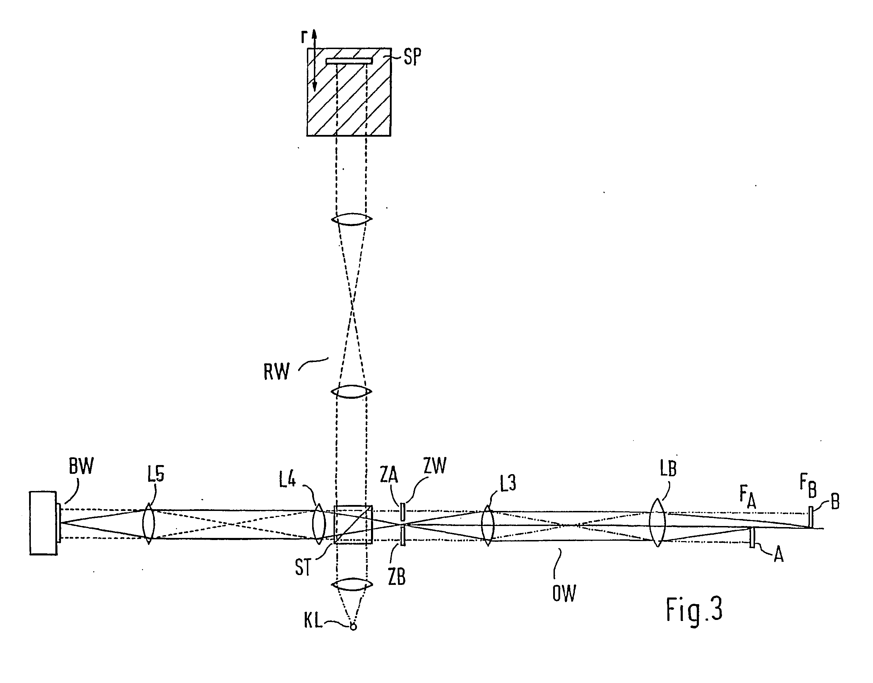

In Fig. 3 ist ein Ausführungsbeispiel der interferometrischen Messvorrichtung gezeigt, bei dem gegenüber der Fig. 2 anstelle der beiden Linsen L1, L2 eine Bifokaloptik LB angeordnet ist, deren Eigenschaft in etwa den beiden Linsen L1, L2 entspricht.

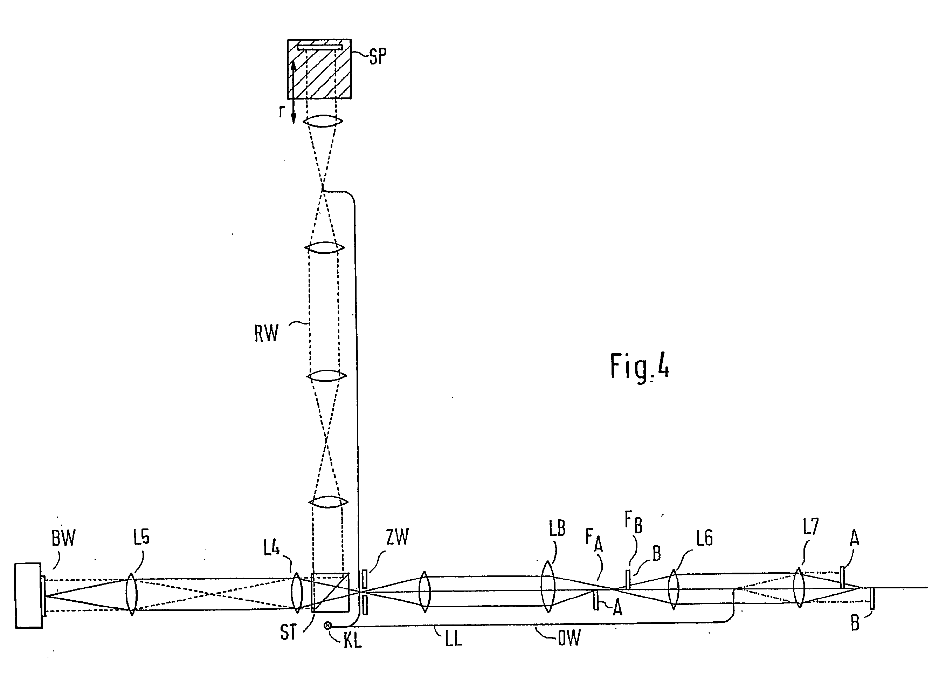

Bei dem in Fig. 4 angegebenen Ausführungsbeispiel sind in den Strahlengang des Objektlichtweges der Bifokaloptik LB objektseitig gelegene weitere Linsen L6, L7 eingebracht. In dem Objektlichtweg OW liegt außerdem ein Lichtwellenleiter LL, über den die kurzkohärente Strahlung von der Strahlungsquelle KL geführt wird, um die Flächen A, B über die weitere Linse L7 mit einer ebenen Wellenfront zu beleuchten. Im Wesentlichen entsprechende Linsen sind auch in den Referenzlichtweg RW zur Kompensation angeordnet, und auch in dem Objektlichtweg wird die Strahlung über einen Lichtwellenleiter zugeführt.

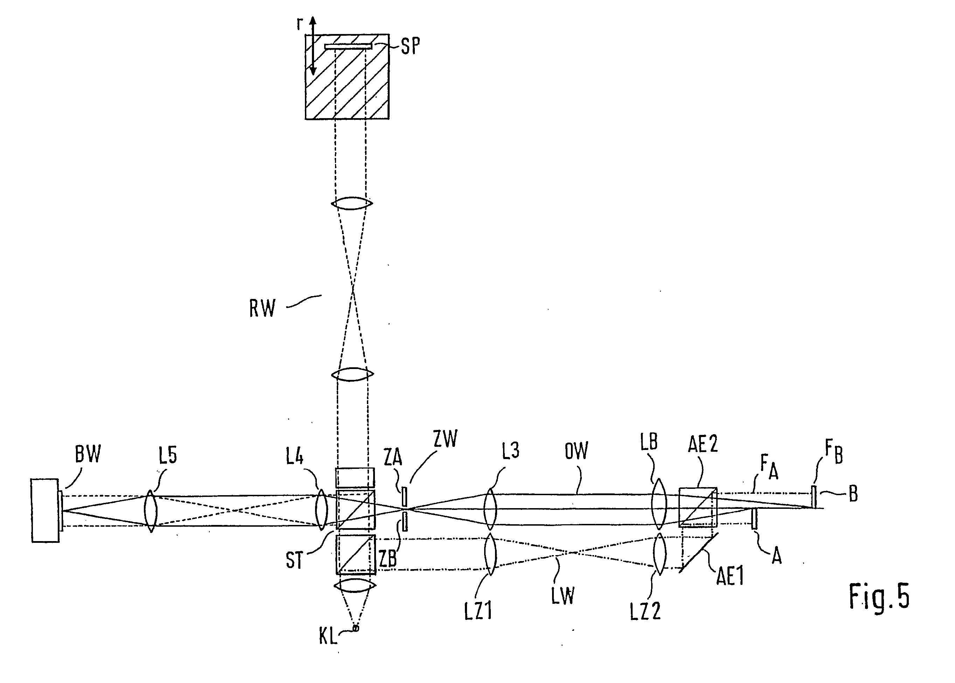

In Fig. 5 ist gegenüber der Fig. 4 in dem Objektlichtweg OW der Lichtwellenleiter LL durch einen Beleuchtungslichtweg LW mit diskreten zusätzlichen Linsen LZ1, LZ2 und Ablenkelementen AE1, AE2 ersetzt, um die Flächen A, B mit einer ebenen Welle zu beleuchten. Die weiteren Linsen L6, L7 sind dabei nicht vorgesehen.

Mit den vorstehend angegebenen interferometrischen Messvorrichtungen werden unter Verwendung von Sonderoptiken in Form der genannten Superpositionsoptiken gleichzeitig die räumlich voneinander getrennten Flächen A, B vermessbar. Dabei können z.B. Abstand bzw. Dicke, Parallelität und Durchmesser der räumlich getrennten Flächen A, B gemessen werden. Die räumlich getrennten Flächen können direkt ober über ein gemeinsames Zwischenbild ZW im Objektlichtweg auf den Bildwandler BW abgebildet werden.

Das gemeinsame Zwischenbild ZW kann direkt oder über eine oder mehrere Zwischenabbildungen im Objektlichtweg auf dem Bildwandler BW z.B. einer CCD-Kamera abgebildet werden.

Der Aufbau der interferometrischen Messvorrichtung ist z.B. als Michelson-Interferometer realisiert. Die kurzkohärente Strahlungsquelle KL ist z.B. eine Superlumineszenzdiode oder eine Leuchtdiode. Mit der Beleuchtung durch die Superpositionsoptik werden die räumlich getrennten Flächen A, B des Objektes beleuchtet, wobei es günstig ist, die getrennten Flächen A, B mit nahezu ebenen Wellen zu beleuchten.

Die Superpositionsoptik in Form der Freie-Segmente-Optik FO kann z.B. aus verschiedenen einzelnen Linsensystemen bestehen, die unterschiedliche Flächen entlang unterschiedlicher optischer Achsen und mit unterschiedlichen optischen Weglängen in die gemeinsame Zwischenbildebene abbilden. Die Freie-Segmente-Optik FO kann mit optischen Elementen, wie z.B. sphärischen Linsen, asphärischen Linsen, Stablinsen oder Grin-Linsen oder mit diffraktiven optischen Elementen oder Prismen oder Spiegeln realisiert werden, die miteinander kombiniert sein können.

Anstelle der Ausbildung der Superpositionsoptik als Bifokaloptik LB kann auch eine Multifokaloptik verwendet werden, wenn mehr Flächen vermessen werden sollen. Die Multifokaloptik kann z.B. mit einer weiteren Linse zu einer telezentrischen Anordnung kombiniert werden.

Zum Abgleich der optischen Weglängen und der Dispersion in beiden Interferometerarmen, nämlich dem Referenzlichtweg RW und dem Objektlichtweg OW, sollten die Faserlängen und Geometrien der verwendeten Lichtwellenleiter möglichst identisch gewählt werden.

Die Superpositionsoptik kann näherungsweise auch durch eine Optik mit großer Schärfentiefe oder mit erweiterter Schärfentiefe, z.B. Axicon, realisiert werden.

Im Falle einer Multifokaloptik bzw. Bifokaloptik als Superpositionsoptik kann zur Kompensation in dem Referenzlichtweg RW eine Optik mit nur einer Brennebene eingesetzt werden, wie aus Fig. 3 ersichtlich.

Auf dem Bildwandler BW wird ein mit der Referenzwelle überlagertes Bild der zu betrachtenden Flächen A, B erzeugt. Zur Datenauswertung erfolgt z.B. eine durch die Abtastbewegung r bewirkte Änderung des Gangunterschiedes zwischen den optischen Weglängen im Objekt- und Referenzlichtweg (Tiefenscan). Es können entsprechend dem Stand der Technik verschiedene Vorgehensweisen zur Änderung des Gangunterschiedes vorgesehen sein, z.B. Bewegung des Referenzspiegels, Bewegung des Objektes in Tiefenrichtung, Bewegung des Objektivs in Tiefenrichtung, Bewegung des gesamten Sensors relativ zu dem Objekt oder auch eine Zwischenbildabtastung oder eine Änderung der optischen Weglänge durch akustooptische Modulatoren.

Im Bild des Objektes tritt hoher Interferenzkontrast dann auf, wenn der Gangunterschied in beiden Interferometerarmen kleiner als die Kohärenzlänge ist. Zur Gewinnung des 3D-Höhenprofils haben sich verschiedene Verfahren etabliert. Sie beruhen darauf, dass während der Tiefenabtastung für jeden Bildpunkt (Pixel) der Gangunterschied detektiert wird, bei welchem der höchste Interferenzkontrast auftritt.

## Patentansprüche

1. Interferometrische Messvorrichtung, die zur Formvermessung eines mehrere Flächen (A, B) aufweisenden Objektes (BO) ausgebildet ist, mit einer eine kurzkohärente Strahlung abgebenden Strahlungsquelle (KL), einem Strahlteiler (ST), mit dem ein über einen Objektlichtweg (OW) zu dem Objekt (BO) geleiteter Objektstrahl und ein über einen Referenzlichtweg (RW) zu einer reflektierenden Referenzebene (SP1) geleiteter Referenzstrahl erzeugbar sind, und mit einem Bildwandler (BW), der die von den Flächen (A, B) und der Referenzebene (SP1) zurückgeworfene und zur Interferenz gebrachte Strahlung aufnimmt und einer Auswerteeinrichtung zum Bestimmen eines die Flächen (A, B) betreffenden Messergebnisses zuführt, wobei für die Messung die optische Länge des Objektlichtweges (OW) relativ zu der optischen Länge des Referenzlichtwegs (RW) änderbar ist und die Messung der Flächen (A, B) unter relativer Änderung der optischen Länge des Objektlichtweges zu der optischen Länge des Referenzlichtweges erfolgt, wobei in dem Objektlichtweg (OW) eine Superpositionsoptik mit einer Multifokaloptik (LB) oder einer Freie-Segmente-Optik (FO) aus verschiedenen Abbildungselementen vorgesehen ist, wobei mit der Superpositionsoptik gleichzeitig von einer ersten Fläche (A) und von mindestens einer weiteren Fläche (B) ein Bild erzeugbar ist, das über mindestens eine Zwischenabbildung im Objektlichtweg auf dem Bildwandler (BW) abbildbar sind.

2. Messvorrichtung nach Anspruch 1,
**dadurch gekennzeichnet,**
**dass** die Freie-Segmente-Optik (FO) zum Aufnehmen von schräg zueinander orientierten Flächen (A, B) ausgebildet ist.

3. interferometrische Messvorrichtung, die zur Formvermessung eines mindestens zwei Flächen (A, B) aufweisenden Objektes (BO) ausgebildet ist, mit einer eine kurzkohärente Strahlung abgebenden Strahlungsquelle (KL), einem Strahlteiler (ST), mit dem ein über einen Objektlichtweg (OW) zu dem Objekt (BO) geleiteter Objektstrahl und ein über einen Referenzlichtweg (RW) zu einer reflektierenden Referenzebene (SP1) geleiteter Referenzstrahl erzeugbar sind, und mit einem Bildwandler (BW), der die von den Flächen (A, B) und der Referenzebene (SP1) zurückgeworfene und zur Interferenz gebrachte Strahlung aufnimmt und einer Auswerteeinrichtung zum Bestimmen eines die Flächen (A, B) betreffenden Messergebnisse zuführt, wobei für die Messung die optische Länge des Objektlichtweges (OW) relativ zu der optischen Länge des Referenzlichtweges (RW) änderbar ist und die Messung der Flächen (A, B) unter relativer Änderung der optischen Länge des Objektlichtweges zu der optischen Länge des Referenzlichtweges erfolgt, wobei in dem Objektlichtweg (OW) eine Abbildungsoptik mit einer Schärfentiefe von mindestens dem optischen Wegunterschied der beiden Flächen vorgesehen ist, mit der gleichzeitig außer von der einen Fläche (A) von mindestens einer davor oder dahinter liegenden, parallelen weiteren Fläche (B) - oder über optische Ablenkelemente schräg oder rechtwinklig zueinander angeordneten Flächen - ein Bild erzeugbar ist, das über mindestens eine im Objektlichtweg erzeugte Zwischenabbildung auf dem Bildwandler (BW) abbildbar ist.

4. Messvorrichtung nach einem der vorhergehenden Ansprüche,
**dadurch gekennzeichnet,**
**dass** der Objektlichtweg (OW) zum Erzeugen eines gemeinsamen Zwischenbildes (ZW), eines Zwischenbildes der Fläche (A) und eines Zwischenbildes der weiteren Fläche (B), in einer gemeinsamen Zwischenbildebene im Objektlichtweg ausgebildet ist und
**dass** das gemeinsame Zwischenbild (ZW) direkt oder über mindestens eine Zwischenabbildung auf dem Bildwandler (BW) abgebildet wird.

5. Messvorrichtung nach Anspruch 4,
**dadurch gekennzeichnet,**
**dass** eine Abtastung des gemeinsamen Zwischenbildes (ZW) erfolgt.

6. Messvorrichtung nach einem der vorhergehenden Ansprüche,
**dadurch gekennzeichnet,**
**dass** der Objektlichtweg (OW) als Endoskop ausgebildet ist.

7. Messvorrichtung nach einem der vorhergehenden Ansprüche,
**dadurch gekennzeichnet,**
**dass** zur Beleuchtung des Objektes (BO, VS) mit einer ebenen Welle ein Lichtwellenleiter (LL) vorgesehen ist, deren objektseitiger Ausgang in eine telezentrische Abbildungsanordnung des Objektlichtweges (OW) gelegt ist, oder dass ein Beleuchtungslichtweg (LW) mit zusätzlichen Linsen (LZ1, LZ2) und Ablenkelementen (AE1, AE2) gebildet ist.

8. Messvorrichtung nach einem der vorhergehenden Ansprüche,
**dadurch gekennzeichnet,**
**dass** der Referenzlichtweg (RW) dem Objektlichtweg (OW) ähnliche oder identische Optiken aufweist.

9. Messvorrichtung nach einem der vorhergehenden Ansprüche,
**dadurch gekennzeichnet,**
**dass** im Objektlichtweg (OW) eine bezüglich des Objektes (30) starre Optik angeordnet ist und
**dass** der starren Optik eine in Richtung ihrer optischen Achse bewegliche Optik (BO) folgt.

10. Messvorrichtung nach Anspruch 9,
**dadurch gekennzeichnet,**
**dass** die starre Optik Teil der Superpositionsoptik ist.

11. Messvorrichtung nach Anspruch 9 oder 10,
**dadurch gekennzeichnet,**
**dass** die starre Optik nach Unendlich abbildet.

12. Messvorrichtung nach einem der Ansprüche 9 bis 11,
**dadurch gekennzeichnet,**
**dass** die starre Optik als Superpositionsoptik ausgebildet ist, mit der mindestens ein zum Objekt (BO) starres Zwischenbild erzeugt wird, und
**dass** als bewegliche Optik eine im Strahlengang hinter dem starren Zwischenbild folgende Objektiv-Optik in Richtung ihrer optischen Achse beweglich zur Abtastung des normal zu dieser Achse ausgerichteten Zwischenbilds in Tiefenrichtung und Abbilden desselben direkt oder über eine oder mehrere Zwischenabbildungen auf dem Bildwandler (BW) ausgebildet ist.

13. Messvorrichtung nach einem der Ansprüche 4 bis 12,
**dadurch gekennzeichnet,**
**dass** die Zwischenabbildung für alle im Zwischenbild (ZW) abgebildeten Objektpunkte gleichen Abbildungsmaßstab besitzt.

14. Messvorrichtung nach einem der Ansprüche 9 bis 13,
**dadurch gekennzeichnet,**
**dass** die starre Optik als 4f-Anordnung ausgebildet ist.

## Claims

1. Interferometric measuring device which is designed for measuring the shape of an object (BO) having a number of surfaces (A, B), having a radiation source (KL) outputting a short coherent radiation, a beam splitter (ST) generating an object beam led via an object light path (OW) to the object (BO) and a reference beam led via a reference light path (RW) to a reflecting reference plane (SP1), and having an image converter (BW) which picks up the radiation retroreflected by the surfaces (A, B) and the reference plane (SP1) and caused to interfere and an evaluation device for determining a measurement result relating to the surfaces (A, B), it being possible for the purpose of measurement to change the optical length of the object light path (OW) relative to the optical length of the reference light path (RW), and the surfaces (A, B) being measured in conjunction with a relative change in the optical length of the object light path relative to the optical length of the reference light path, a superposition optics with a multifocal optics (LB) or a free-segment optics (FO) composed of various imaging elements being provided in the object light path (OW), it being possible to use the superposition optics to generate simultaneously an image of a first surface (A) and of at least one further surface (B), which can be imaged on the image converter (BW) via at least one intermediate image in the object light path.

2. Measuring device according to Claim 1, **characterized in that** the free-segment optics (FO) is designed for making up surfaces (A, B) orientated obliquely to one another.

3. Interferometric measuring device which is designed for measuring the shape of an object (BO) having at least two surfaces (A, B), having a radiation source (KL) outputting a short coherent radiation, a beam splitter (ST) generating an object beam led via an object light path (OW) to the object (BO) and a reference beam led via a reference light path (RW) to a reflecting reference plane (SP1), and having an image converter (BW) which picks up the radiation retroreflected by the surfaces (A, B) and the reference plane (SP1) and caused to interfere and an evaluation device for determining a measurement result relating to the surfaces (A, B), it being possible for the purpose of measurement to change the optical length of the object light path (OW) relative to the optical length of the reference light path (RW), and the surfaces (A, B) being measured in conjunction with a relative change in the optical length of the object light path relative to the optical length of the reference light path, there being provided in the object light path (OW) an imaging optics with a depth of field of at least the optical path difference of the two surfaces with the aid of which, apart from an image of one surface (A), it is possible simultaneously to generate an image of at least one parallel further surface (B) lying upstream or downstream of said surface (A) - or, via optical deflecting elements an image of surfaces arranged obliquely or at right angles to one another - which image can be imaged on the image converter (BW) via at least one intermediate image generated in the object light path.

4. Measuring device according to one of the preceding claims, **characterized in that** in order to generate a common intermediate image (ZW), an intermediate image of the surface (A) and an intermediate image of the further surface (B), the object light path (OW) is imaged in a common intermediate image plane in the object light path, and **in that** the common intermediate image (ZW) is imaged on the image converter (BW) directly or via at least one instance of intermediate imaging.

5. Measuring device according to Claim 4, **characterized in that** the common intermediate image (ZW) is scanned.

6. Measuring device according to one of the preceding claims, **characterized in that** the object light path (OW) is designed as an endoscope.

7. Measuring device according to one of the preceding claims, **characterized in that** in order to illuminate the object (BO, VS) with a plane wave, an optical waveguide (LL) is provided whose object-side output is put into a telecentric imaging arrangement of the object light path (OW), or **in that** an illumination light path (LW) is formed with additional lenses (LZ1, LZ2) and deflecting elements (AE1, AE2).

8. Measuring device according to one of the preceding claims, **characterized in that** the reference light path (RW) has optics which are similar or identical to the object light path (OW).

9. Measuring device according to one of the preceding claims, **characterized in that** an optics which is rigid with reference to the object (30) is arranged in the object light path (OW), and **in that** the rigid optics is followed by an optics (BO) which can be moved in the direction of its optical axis.

10. Measuring device according to Claim 9, **characterized in that** the rigid optics is part of the superposition optics.

11. Measuring device according to Claim 9 or 10, **characterized in that** the rigid optics images at infinity.

12. Measuring device according to one of Claims 9 to 11, **characterized in that** the rigid optics is designed as a superposition optics with the aid of which at least one intermediate image which is rigid relative to the object (BO) is generated, and **in that** the moveable optics is designed as an objective optics which follows in the beam path downstream of the rigid intermediate image and can be moved in the direction of its optical axis in order to scan the intermediate image, aligned normal to this axis, in the depth direction and to image this intermediate image on the image converter (BW) directly or via one or more intermediate images.

13. Measuring device according to one of Claims 4 to 12, **characterized in that** the intermediate image has the same linear magnification for all the object points imaged in the intermediate image (ZW).

14. Measuring device according to one of Claims 9 to 13, **characterized in that** the rigid optics is designed as a 4f arrangement.

## Revendications

1. Dispositif de mesure interférométrique destiné à mesurer la forme d'un objet (BO) ayant plusieurs surfaces (A, B), et comprenant :
une source de rayonnement (KL) fournissant un rayonnement cohérent court, un guide de lumière (ST) pour générer un faisceau objet suivant un chemin de lumière objet (OW) vers l'objet (BO) et un faisceau de référence passant par un chemin de lumière de référence (RW) vers un plan de référence (SP1) réfléchissant, et
un convertisseur d'image (BW) qui reçoit le rayonnement renvoyé par les surfaces (A, B) et le plan de référence (SP1) pour les faire interférer ainsi qu'une installation d'exploitation pour déterminer le résultat de la mesure concernant ces surfaces (A, B),
la mesure de la longueur optique du chemin de la lumière objet (OW) par rapport à la longueur optique du chemin de lumière de référence (RW) étant variable et la mesure des surfaces (A, B) se faisant sous une variation relative de la longueur optique du chemin de la lumière objet par rapport à la longueur optique du chemin de lumière de référence,
selon lequel le chemin de lumière objet (OW) comporte une optique de superposition avec une optique multifocale (LB) ou une optique à segment libre (FO) composée de plusieurs éléments formant des images,
l'optique de superposition générant en même temps une image d'une première surface (A) et d'au moins une autre surface (B), qui est transmise par au moins une image intermédiaire dans le champ de lumière objet sur le convertisseur d'image (BW).

2. Dispositif de mesure selon la revendication 1,
**caractérisé en ce que**
l'optique à segments libres (FO) est conçue pour détecter des surfaces (A, B) orientées en biais les unes par rapport aux autres.

3. Dispositif de mesure interférométrique destiné à mesurer la forme d'un objet (BO) ayant plusieurs surfaces (A, B), et comprenant :
une source de rayonnement (KL) fournissant un rayonnement cohérent court, un guide de lumière (ST) pour générer un faisceau objet suivant un chemin de lumière objet (OW) vers l'objet (BO) et un faisceau de référence passant par un chemin de lumière de référence (RW) vers un plan de référence (SP1) réfléchissant, et
un convertisseur d'image (BW) qui reçoit le rayonnement renvoyé par les surfaces (A, B) et le plan de référence (SP1) pour les faire interférer ainsi qu'une installation d'exploitation pour déterminer le résultat de la mesure concernant ces surfaces (A, B),
la mesure de la longueur optique du chemin de la lumière objet (OW) par rapport à la longueur optique du chemin de lumière de référence (RW) étant variable et la mesure des surfaces (A, B) se faisant sous une variation relative de la longueur optique du chemin de la lumière objet par rapport à la longueur optique du chemin de lumière de référence, selon lequel
le chemin de lumière (OW) comporte une optique de reproduction avec une profondeur de champ correspondant au moins à la différence de chemins optiques des deux surfaces, générant une image en même temps non seulement de l'une des surfaces (A) mais également d'au moins une autre surface (B), parallèle à celle-ci et située devant ou derrière (ou par des éléments de déviation optique des surfaces en biais ou à l'équerre les unes par rapport aux autres),
image reproduite sur le convertisseur d'image (BW) par au moins une image intermédiaire dans le chemin de la lumière objet.

4. Dispositif de mesure selon l'une des revendications précédentes,
**caractérisé en ce que**
le chemin de lumière objet (OW) est réalisé pour générer une image intermédiaire commune (ZW), une image intermédiaire de la surface (A) et une image intermédiaire de l'autre surface (B), dans un plan commun d'images intermédiaires dans le chemin de la lumière objet et
l'image intermédiaire commune (ZW) est reproduite sur le convertisseur d'image (BW), soit directement, soit avec au moins une image intermédiaire.

5. Dispositif de mesure selon la revendication 4,
**caractérisé en ce qu'**
on détecte l'image intermédiaire commune (ZW).

6. Dispositif de mesure selon l'une quelconque des revendications précédentes,
**caractérisé en ce que**
le chemin de la lumière objet (OW) est un endoscope.

7. Dispositif de mesure selon l'une quelconque des revendications précédentes,
**caractérisé par**
un guide de lumière (LL) pour éclairer l'objet (BO, VS) avec une onde plane, dont la sortie côté objet du guide de lumière est située dans un dispositif de reproduction télécentrique du chemin de lumière objet (OW), ou un chemin de lumière d'éclairage (LW) avec des lentilles supplémentaires (LZ1, LZ2) et des éléments déflecteurs (AE1, AE2).

8. Dispositif de mesure selon l'une quelconque des revendications précédentes,
**caractérisé en ce que**
le chemin de lumière de référence (RW) comporte des optiques analogues ou identiques à celles du chemin de lumière objet (OW).

9. Dispositif de mesure selon l'une quelconque des revendications précédentes,
**caractérisé en ce que**
le chemin de lumière objet (OW) comporte une optique fixe par rapport à l'objet (30) et
l'optique fixe est suivie par une optique mobile (BO) dans la direction de son axe optique.

10. Dispositif de mesure selon la revendication 9,
**caractérisé en ce que**
l'optique fixe fait partie de l'optique de superposition.

11. Dispositif de mesure selon les revendications 9 ou 10,
**caractérisé en ce que**
l'optique fixe rejette l'image à l'infini.

12. Dispositif de mesure selon l'une des revendications 9 à 11,
**caractérisé en ce que**
l'optique fixe est une optique de superposition générant au moins une image intermédiaire fixe par rapport à l'objet (BO), et
l'optique mobile est une optique d'objectif placée derrière l'image intermédiaire fixe dans le chemin du faisceau pour détecter l'image intermédiaire normale à cet axe dans la direction de la profondeur et reproduire celle-ci directement ou par une ou plusieurs images intermédiaires sur le convertisseur d'image (BW).

13. Dispositif de mesure selon l'une des revendications 4 à 12,
**caractérisé en ce que**
l'image intermédiaire a la même échelle de reproduction pour tous les points objets reproduits dans l'image intermédiaire (ZW).

14. Dispositif de mesure selon l'une des revendications 9 à 13,
**caractérisé en ce que**
l'optique fixe est un système optique à 4 foyers.
